# EUROPEAN PATENT APPLICATION

(11) **EP 4 704 024 A1**
(43) Date of publication of application: **04.03.2026**
(21) Application number: 24796744.1
(22) Date of filing: 04.04.2024
(51) Int. Cl.: G06Q 50/22, G06Q 10/0639, G16H 40/00

(54) **WORK DIVISION INFERENCE METHOD, WORK DIVISION INFERENCE PROGRAM, AND INFORMATION PROCESSING DEVICE**

(30) Priority: 28.04.2023 JP 2023075059
(71) Applicant: National Institute of Advanced Industrial Science and Technology, Chiyoda-ku Tokyo 100-8921 (JP)
(72) Inventor: MIWA, Hiroyasu, Tsukuba-shi, Ibaraki 305-8561 (JP)
(74) Representative: Scott, Stephen John
(86) International application number: PCT/JP2024/013988
(87) International publication number: WO 2024/224986

(57) **Abstract**

A task category estimation method of estimating the category of a task performed by a caregiver at a caregiving site includes acquiring location information regarding a plurality of locations to which the caregiver moved at the caregiving site and time information regarding a time that the caregiver stayed at each of the plurality of locations to which the caregiver moved, estimating a category of a task performed by the caregiver at at least one location among the plurality of locations, based on the location information and the time information, and outputting an estimated result of the category of the task performed by the caregiver.

## Description

### TECHNICAL FIELD

The present disclosure relates to a task category estimation method, a task category estimation program, and an information processing apparatus that are related to caregiving.

### BACKGROUND

Conventionally, a method of using an apparatus to record task data collected in a time and motion study is known (see, for example, Patent Literature (PTL) 1).

### CITATION LIST

### Patent Literature

PTL 1: JP 2003-280726 A

### SUMMARY

### (Technical Problem)

Even when task data collected in a time and motion study is recorded using an apparatus, an observer must accompany the subject to identify task categories for the subject and collect the task data. Furthermore, collecting data on caregiving is useful for improving tasks related to caregiving. Demand exists for easy collection of task data related to caregiving.

An aim of the present disclosure is to provide a task category estimation method, a task category estimation program, and an information processing apparatus that can conveniently collect task data related to caregiving.

### (Solution to Problem)

(1) A task category estimation method according to one embodiment of the present disclosure is a task category estimation method of estimating a category of a task performed by a caregiver at a caregiving site, the task category estimation method including acquiring, by an information processing apparatus, location information regarding a plurality of locations to which the caregiver moved at the caregiving site and time information regarding a time that the caregiver stayed at each of the plurality of locations to which the caregiver moved; estimating, by the information processing apparatus, a category of a task performed by the caregiver at at least one location among the plurality of locations to which the caregiver moved, based on the location information and the time information; and outputting an estimated result of the category of the task performed by the caregiver.
(2) In the task category estimation method of (1), in the acquiring of the time information, the time that the caregiver stayed at each of the plurality of locations may be acquired using a sensor.
(3) In the task category estimation method of (1) or (2), the time information may include a length of time that the caregiver stayed at at least one of the plurality of locations.
(4) In the task category estimation method of any one of (1) to (3), the location information for the caregiver may include a category of a task that the caregiver can perform at at least one of the plurality of locations.
(5) In the task category estimation method of any one of (1) to (4), the location information for the caregiver may include information on equipment installed in at least one of the plurality of locations.
(6) In the task category estimation method of any one of (1) to (5), the location information for the caregiver may include information on a location at which the caregiver stayed before the location at which the caregiver performed the task to be estimated.
(7) In the task category estimation method of any one of (1) to (6), the location information for the caregiver may include information on a location at which the caregiver stayed after the location at which the caregiver performed the task to be estimated.
(8) In the task category estimation method of any one of (1) to (7), in the estimating of the category of the task performed by the caregiver, the information processing apparatus may input the location information and the time information into a task category estimation model, and acquire an estimated result, outputted from the task category estimation model, of the category of the task performed by the caregiver.
(9) The task category estimation method of (8) may further include performing, by the information processing apparatus, learning using training data including the location information and the time information, and ground truth data including categories of tasks performed by the caregiver, to generate the task category estimation model.
(10) In the task category estimation method of any one of (1) to (9), in the outputting of the estimated result of the category of the task performed by the caregiver, the information processing apparatus may display the estimated result of the category of the task performed by the caregiver in a form of a timeline corresponding to a time period during which the caregiver performed the task.
(11) In the task category estimation method of any one of (1) to (10), in the outputting of the estimated result of the category of the task performed by the caregiver, the information processing apparatus may output statistical data on the category of the task performed by the caregiver.
(12) In the task category estimation method of any one of (1) to (11), the task performed by the caregiver may be classified into at least two levels including a first level and a second level below the first level, and in the estimating of the category of the task performed by the caregiver, the information processing apparatus may estimate the category of the task based on at least one of classification into the first level and classification into the second level.
(13) In the task category estimation method of (12), in the estimating of the category of the task performed by the caregiver, the information processing apparatus may estimate the category of the task based on both classification into the first level and classification into the second level.
(14) In the task category estimation method of (12) or (13), task performed by the caregiver may be classified into the first level by type of care service, and the task performed by the caregiver may be classified into the second level by purpose or situation.
(15) In the task category estimation method of (14), the task performed by the caregiver may be further classified into a third level below the second level, and the tasks performed by the caregiver may be classified into the third level by content of the task performed by the caregiver.
(16) A task category estimation program according to an embodiment of the present disclosure is configured to cause an information processing apparatus to execute the task category estimation method of any one of (1) to (15).
(17) An information processing apparatus according to an embodiment of the present disclosure includes a processor configured to execute the task category estimation method of any one of (1) to (15).

### (Advantageous Effect)

The task category estimation method, task category estimation program, and information processing apparatus according to an embodiment of the present disclosure can conveniently collect task data related to caregiving.

### BRIEF DESCRIPTION OF THE DRAWINGS

In the accompanying drawings:
FIG. 1 is a block diagram illustrating an example configuration of an information processing system according to the present disclosure;
FIG. 2 is a block diagram illustrating an example configuration of an action learning unit;
FIG. 3 is a block diagram illustrating an example configuration of a task category estimation unit;
FIG. 4 is a diagram illustrating an example of a hierarchical structure of task categories;
FIG. 5A is a diagram illustrating an independent process;
FIG. 5B is a diagram illustrating a parallel process in which some actions are parallel;
FIG. 5C is a diagram illustrating a parallel process in which initial actions are parallel;
FIG. 5D is a diagram illustrating a parallel process in which all actions are parallel;
FIG. 5E is a diagram illustrating a parallel process in which final actions are parallel;
FIG. 5F illustrates an interrupt process;
FIG. 6 is a timeline illustrating time periods during which a caregiver is estimated to have performed tasks in each category;
FIG. 7 is a timeline illustrating the time periods during which a caregiver stayed at each location;
FIG. 8 is a bar graph illustrating the estimated length of time that a caregiver performed tasks in each category;
FIG. 9 is a flowchart illustrating an example of a procedure for generating a task category estimation model; and
FIG. 10 is a flowchart illustrating an example of the steps of a task category estimation method according to the present disclosure.

### DETAILED DESCRIPTION

A shortage of caregivers is becoming a problem in the field of care services. There is a need to improve productivity so that work can be carried out efficiently with a small number of personnel. A common method for improving productivity in care service sites is to understand the care service process, which is a series of steps at the care service site, and to implement measures such as technology introduction or process improvement.

Here, in order to understand the care service process, it is necessary to collect data on the tasks of caregivers. The tasks of a caregiver each fall into one of several categories. The task data includes information on the area where the caregiver performed tasks of each category, or the time or frequency with which the caregiver performed tasks of each category. Task data may be collected through time studies in which tasks are visually observed by humans. However, the collection of task data is a heavy burden. It is necessary to collect task data efficiently.

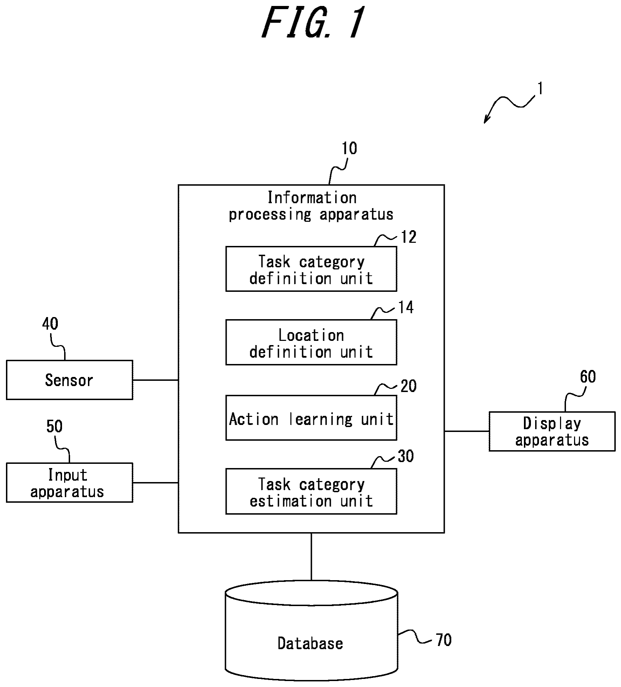

Therefore, the present disclosure describes an information processing system 1 (see FIG. 1) that has been developed to enable efficient collection of task data on caregivers. The information processing system 1 according to the present disclosure has been developed with a focus on the fact that care services are provided as a series of steps, and that there are actions that are strongly linked to the area where the task is performed. The information processing system 1 of the present disclosure estimates what tasks a caregiver performed based on a plurality of locations to which the caregiver moved and the time that the caregiver stayed at each location, and outputs the estimated result of the tasks. The information processing system 1 according to the present disclosure will be described below with reference to the drawings.

### (Overview of information processing system 1)

As illustrated in FIG. 1, an information processing system 1 according to an embodiment includes an information processing apparatus 10, a sensor 40, an input apparatus 50, a display apparatus 60, and a database 70. The information processing apparatus 10 acquires information regarding locations to which a caregiver moved in a caregiving site such as a care facility, and information regarding the time that the caregiver stayed at each location. The information regarding the location of the caregiver is also referred to as location information. The information regarding the time that the caregiver stayed at each location is also referred to as time information. The location information may include locations to which the caregiver moved and stayed, or locations through which the caregiver passed while moving. In other words, the location information may include the movement history of the caregiver to a plurality of locations. The time information may include the length of time that the caregiver has stayed at each location in the past, or the time that the caregiver's stay began and ended at each location in the past. The time when the caregiver starts staying at a certain location corresponds to the time when the caregiver completes moving to that location. The time at which the caregiver finishes staying at a certain location corresponds to the time at which the caregiver starts moving from that location. The location information and time information may be generated based on the measurement results from the sensor 40, as described below.

The information processing apparatus 10 estimates the caregiving-related tasks performed by the caregiver based on the location information and the time information for the caregiver. Additionally, caregiving-related tasks may be classified into a plurality of categories, as described below. Caregiving-related tasks may include tasks that are performed by a caregiver while moving to a plurality of locations. In other words, one task performed by the caregiver may be a task that combines actions that the caregiver performs at each of a plurality of locations. One task performed by a caregiver may be a task that combines the caregiver's movement between a plurality of locations and an action that the caregiver performs at each of the plurality of locations. The information processing apparatus 10 may estimate the category of caregiving-related tasks performed by the caregiver.

The information processing apparatus 10 may display, on the display apparatus 60, a timeline of the tasks of each category that are estimated to have been performed by the caregiver. The information processing apparatus 10 may record the duration or frequency of the tasks of each category that are estimated to have been performed by the caregiver and display the recorded results on the display apparatus 60.

### (Configuration example of information processing system 1)

An example configuration of the information processing system 1 will be described below.

### <Information processing apparatus 10>

As illustrated in FIG. 1, the information processing apparatus 10 includes a task category definition unit 12, a location definition unit 14, an action learning unit 20, and a task category estimation unit 30. The task category definition unit 12 defines the categories of caregiving-related tasks performed by the caregiver. The location definition unit 14 defines locations to which the caregiver moved and stayed, locations through which the caregiver passed while moving, or locations at which the caregiver performed a task (or a task related to caregiving). The action learning unit 20 generates a task category estimation model used to estimate the category of caregiving-related tasks performed by the caregiver. The task category estimation unit 30 uses the task category estimation model to estimate the category of caregiving-related tasks performed by the caregiver.

### <<Action learning unit 20>>

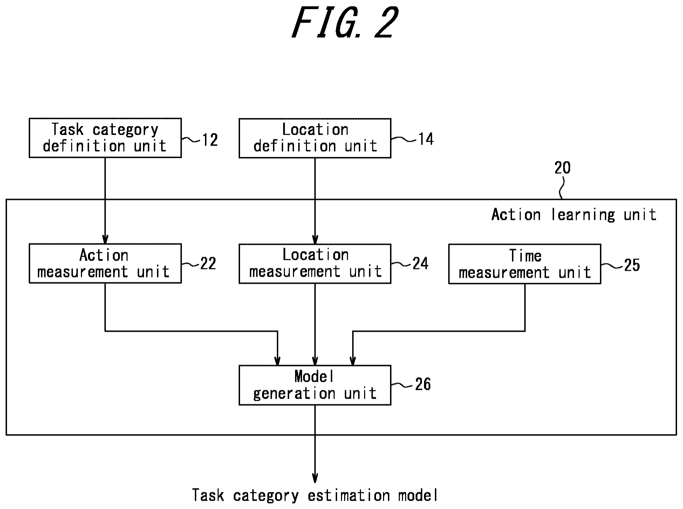

As illustrated in FIG. 2, the action learning unit 20 includes an action measurement unit 22, a location measurement unit 24, a time measurement unit 25, and a model generation unit 26.

Each component of the action learning unit 20 may be configured to include one or more processors. The processor may include, but is not limited to, a general-purpose processor or a dedicated processor specialized for a particular process. Each component of the action learning unit 20 may be configured to include one or more dedicated circuits. The dedicated circuit may include, for example, a field-programmable gate array (FPGA) or an application specific integrated circuit (ASIC). Each component of the action learning unit 20 may be configured to include a dedicated circuit instead of a processor, or may be configured to include a dedicated circuit together with a processor. Each of the components of the action learning unit 20 may be configured as a separate entity. At least some of the components of the action learning unit 20 may be configured as an integrated unit.

The action learning unit 20 may be configured to include a memory unit. The memory unit may store programs executed by each component of the action learning unit 20 or information, data, or the like used in the processing of each component. The memory unit may be configured to include, for example, a semiconductor memory, a magnetic memory, or an optical memory. The memory unit may be configured to include an electromagnetic storage medium such as a magnetic disk. The memory unit may be configured to include a non-transitory computer readable medium. The memory unit may function as, for example, a main storage device, an auxiliary storage device, or a cache memory. The memory unit may function as a working memory for each component of the action learning unit 20. The memory unit may be configured integrally with the processor of the action learning unit 20, or may be configured separately from the processor of the action learning unit 20.

### <<Task category estimation unit 30>>

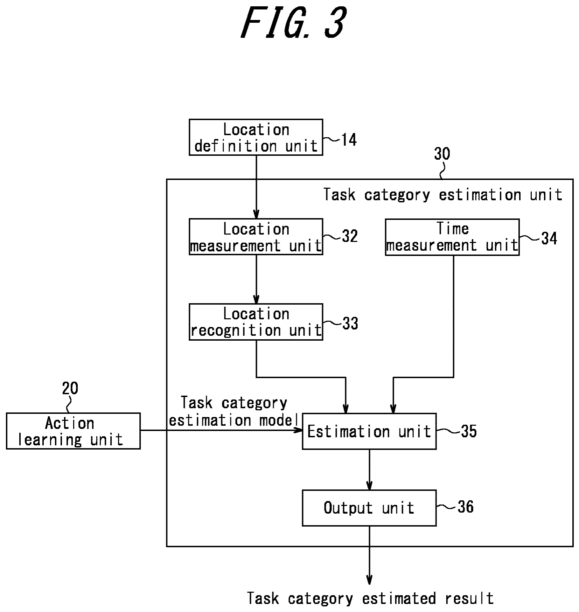

As illustrated in FIG. 3, the task category estimation unit 30 includes a location measurement unit 32, a location recognition unit 33, a time measurement unit 34, an estimation unit 35, and an output unit 36.

Each component of the task category estimation unit 30, like each component of the action learning unit 20, may be configured to include one or more processors or one or more dedicated circuits. The task category estimation unit 30 may be configured to include a memory unit, like the action learning unit 20. The action learning unit 20 and the task category estimation unit 30 may be configured as separate units or may be configured integrally.

### <<Other configurations of the information processing apparatus 10>>

The information processing apparatus 10 may be configured to include a communication module configured to be capable of communicating with other devices such as the sensor 40, the input apparatus 50, the display apparatus 60, or the database 70. The communication module may be configured to be capable of communicating with other devices via a network or may be configured to be capable of communicating with other devices in a P2P (Peer to Peer) manner without using a network. The communication module may be configured to communicate with other devices in a wired or wireless manner. The communication module may be compatible with mobile communication standards such as 4G (4th Generation) or 5G (5th Generation). The communication module may be compatible with communication standards such as LAN (Local Area Network). The communication module may support wired or wireless communication standards. The communication module is not limited to these and may be compatible with various communication standards.

The information processing apparatus 10 may be configured to include at least one server. The information processing apparatus 10 may be realized as a computer such as a desktop PC (Personal Computer), a notebook PC, or a tablet PC. The information processing apparatus 10 may be realized as a smartphone, a tablet, or the like. The information processing apparatus 10 is not limited to these examples and may include various devices.

The information processing apparatus 10 may be configured separately into an apparatus including the action learning unit 20 and an apparatus including the task category estimation unit 30. The information processing apparatus 10 including the action learning unit 20 is also referred to as a learning apparatus. The information processing apparatus 10 including the task category estimation unit 30 is also referred to as a task category estimation apparatus.

### <Sensor 40>

The sensor 40 is configured to be able to detect the location of a caregiver in a caregiving site such as a care facility. The sensor 40 outputs the detection result of the location of the caregiver to the information processing apparatus 10. The sensor 40 may include, for example, a location measuring device that is installed at the caregiving site, uses radio waves for wireless communication, and detects the location information for the caregiver. The sensor 40 may include a human presence sensor or a camera installed at an area where the location of the caregiver is to be detected, so that the location of the caregiver in the caregiving site can be detected. The sensor 40 may include a location measuring device that uses a satellite positioning system such as the Global Positioning System (GPS). The sensor 40 may include a location measuring device that utilizes pedestrian dead reckoning (PDR). The sensor 40 is not limited to these examples and may include a variety of other devices.

The sensor 40 may be configured to detect the body movement of the caregiver. The sensor 40 may include, for example, an acceleration sensor worn by the caregiver. The sensor 40 may include, for example, a depth camera that captures an image of the caregiver and generates point cloud data on the caregiver. The sensor 40 is not limited to these examples and may include a variety of other devices.

### <Input apparatus 50>

The input apparatus 50 accepts input of information. The input apparatus 50 may include, for example, a touch sensor or a pointing device such as a mouse. The input apparatus 50 may include physical keys. The input apparatus 50 may include a voice input device such as a microphone. The input apparatus 50 may include a display apparatus that displays the contents of the received input. The input apparatus 50 may be configured as a touch display in which a display apparatus and a touch panel are integrated.

The input apparatus 50 may be realized, for example, as a computer such as a desktop PC, a notebook PC, or a tablet PC, or as a terminal such as a smartphone or a tablet. The input apparatus 50 is not limited to these examples and may be realized as various devices.

### <Display apparatus 60>

The display apparatus 60 displays information such as information generated by the information processing apparatus 10. The display apparatus 60 may be configured to include any of various displays, such as a liquid crystal display, an organic EL (Electro-Luminescence) or inorganic EL display, or an LED (Light Emitting Diode) display, so as to display information as characters, symbols, images, or the like.

The display apparatus 60 may be realized, for example, as a computer such as a desktop PC, a notebook PC, or a tablet PC, or as a terminal such as a smartphone or a tablet. The display apparatus 60 is not limited to these examples and may be realized as various devices.

### <Database 70>

The database 70 may store the location information and time information for the caregiver as collected by the information processing apparatus 10. The database 70 may store the estimated results of the categories of tasks of caregivers as estimated by the information processing apparatus 10.

The database 70 may be configured to include at least one server or storage apparatus. The database 70 may be configured to include, for example, a semiconductor memory, a magnetic memory, or an optical memory. The database 70 may be configured to include an electromagnetic storage medium such as a magnetic disk. The database 70 may be configured to include a communication module configured to be capable of communicating with other devices such as the information processing apparatus 10.

The information processing system 1, the information processing apparatus 10, or the database 70 described above may be realized using a cloud service or in an on-premises environment.

### (Example of operations for estimating category of task)

An example of an operation in which the information processing apparatus 10 infers the category of the task performed by the caregiver in the information processing system 1 will be described below.

The information processing apparatus 10 uses the task category definition unit 12 to define categories of tasks to be estimated. The task category definition unit 12 defines a plurality of categories into which caregiving-related tasks performed by a caregiver are classified. The caregiving-related tasks performed by the caregiver may be classified, for example, by type of care service. Examples of categories for classifying caregiving-related tasks performed by the caregiver are described below. The information processing apparatus 10 need not include the task category definition unit 12 and may instead acquire definitions of categories for classifying caregiving-related tasks performed by the caregiver from an external apparatus.

As described above, the information processing apparatus 10 estimates the category of the caregiving-related task performed by the caregiver based on the location to which the caregiver moves when performing the task. Therefore, the information processing apparatus 10 uses the location definition unit 14 to define the location to which the caregiver moves. The location definition unit 14 defines the location to which the caregiver moves. The location to which the caregiver moves may include a location at which the caregiver stays or a location through which the caregiver passes. The locations to which the caregiver moves may be defined as areas within the care facility that are classified by the function of providing care services. The locations to which the caregiver moves may be defined as meta information such as the care service resident's rooms, eating places, bathrooms, toilets, the recreation room, or the hallway, for example. The location to which the caregiver moves may be defined as coordinate information that can be associated with coordinates of a floor plan within the care facility. The information processing apparatus 10 need not include the location definition unit 14 and may instead acquire the definition of the location to which the caregiver moves from an external apparatus.

### <Example of operation of action learning unit 20>

An example of an operation in which the action learning unit 20 of the information processing apparatus 10 generates a task category estimation model will be described below with reference to FIG. 2. Caregiving-related tasks will hereinafter be referred to simply as tasks.

The action measurement unit 22 acquires information on the actions of the caregiver. The actions of the caregiver may be measured by the sensor 40. The sensor 40 may measure the actions of the caregiver by detecting the movements of various parts of the caregiver's body. When an action by the caregiver is measured by the sensor 40, the action measurement unit 22 may acquire information regarding the action by the caregiver from the sensor 40.

The action measurement unit 22 acquires the definition of the categories of tasks performed by the caregiver from the task category definition unit 12. The action measurement unit 22 associates information about the action by the caregiver with at least one of a plurality of categories of tasks performed by the caregiver. The category of a task performed by the caregiver may be associated with information regarding an action by the caregiver as a result of the caregiver himself, or another worker, inputting the category to the input apparatus 50.

The action measurement unit 22 outputs the category of the task performed by the caregiver and associated with the information on the action by the caregiver to the model generation unit 26.

The location measurement unit 24 acquires the definition of the location to which the caregiver moves from the location definition unit 14. The location measurement unit 24 acquires the location information for the caregiver. The location measurement unit 24 may acquire meta information that defines the location to which the caregiver moves as the location information for the caregiver. The location measurement unit 24 may acquire coordinate information for the caregiver, convert the coordinate information into meta information, and acquire the meta information as the location information for the caregiver. In other words, the location information for the caregiver may include meta information that defines the location to which the caregiver moves. The location information for the caregiver may include coordinate information for the caregiver. The location information for the caregiver may include categories of tasks that can be performed at each location to which the caregiver can move. The location information for the caregiver may include information about the equipment installed at each location to which the caregiver can move.

The location of the caregiver may be measured by the sensor 40. The sensor 40 may measure the location of the caregiver, for example, by detecting the location of the caregiver as coordinates. The sensor 40 may detect the presence of a caregiver at a specific area in a caregiving site, such as a care facility, and measure the area where the presence of the caregiver is detected as the location of the caregiver. In a case in which the location of the caregiver is measured by the sensor 40, the location measurement unit 24 may acquire the location information for the caregiver from the sensor 40.

The location of the caregiver may be measured by the caregiver himself or another worker inputting the location into the input apparatus 50. In a case in which the location of the caregiver is input to the input apparatus 50, the location measurement unit 24 may acquire the location information for the caregiver from the input apparatus 50.

The location measurement unit 24 outputs the location information for the caregiver to the model generation unit 26 as the location to which the caregiver moved.

The caregiver may move to a plurality of locations. The location measurement unit 24 may output information about a plurality of locations to which the caregiver moved to the model generation unit 26 as the location information for the caregiver. The action measurement unit 22 acquires information about actions of the caregiver at at least one of the plurality locations to which the caregiver moved. In a case in which the action measurement unit 22 acquires information about the action of the caregiver at one location, the action measurement unit 22 associates the information about the action of the caregiver at that location with the category of task performed by the caregiver at that location. In a case in which the action measurement unit 22 acquires information about the actions of the caregiver at two or more locations, the action measurement unit 22 associates the information about the action of the caregiver at each location with the category of task performed by the caregiver at each location. The action measurement unit 22 outputs, to the model generation unit 26, information in which the category of the task performed by the caregiver is associated with information related to the action of the caregiver at each location.

The location information for the caregiver may include information on the location at which the caregiver stayed before the location for which the action measurement unit 22 acquired information on the action by the caregiver. The location information for the caregiver may include information on the location to which the caregiver moved after the location for which the action measurement unit 22 acquired information on the action by the caregiver. The location information for the caregiver may include a category of tasks that can be performed at at least one of a plurality of locations to which the caregiver moved. The location information for the caregiver may include information on equipment installed in at least one of the plurality of locations to which the caregiver moved.

The time measurement unit 25 acquires time information for the caregiver. In a case in which the caregiver moved to one location, the time information includes the time that the caregiver stayed at that location. In a case in which the caregiver moved to a plurality of locations, the time information includes the time that the caregiver stayed at each location. The time that a caregiver stayed at a certain location may be expressed as the length of time that the caregiver stayed at that location. The time that a caregiver stayed at a certain location may be represented by at least one of, or a combination of, the time that the caregiver started staying at that location and the time that the caregiver finished staying at that location.

The time that the caregiver stayed in a certain location may be measured by the sensor 40 as described below. In a case in which the time that the caregiver stayed at a certain location is measured by the sensor 40, the time measurement unit 25 may acquire the time information for the caregiver from the sensor 40. In this manner, the sensor 40 may be provided with a time measurement function. The time measurement unit 25 may use the sensor 40 to acquire the time that the caregiver stayed at one location or at each of a plurality of locations.

The time that a caregiver stayed at a certain location may be measured by the caregiver himself or another worker inputting the time into the input apparatus 50. In a case in which the time that the caregiver stayed at a certain location is inputted to the input apparatus 50, the time measurement unit 25 may acquire the time information for the caregiver from the input apparatus 50.

The time measurement unit 25 outputs the time information for the caregiver to the model generation unit 26 as the time that the caregiver stayed at one location or at each of a plurality of locations to which the caregiver moved.

In a case in which the caregiver moves to a plurality of locations, the time measurement unit 25 may output information regarding the time that the caregiver stayed at each of the plurality of locations to the model generation unit 26 as the time information. The time information for the caregiver may include information on the time that the caregiver stayed at a location at which the caregiver stayed before the location for which the action measurement unit 22 acquired information on the action by the caregiver. The time information for the caregiver may include information on the time that the caregiver stayed at a location to which the caregiver moved after the location for which the action measurement unit 22 acquired information on the action by the caregiver.

In a case in which the caregiver moved to one location, the model generation unit 26 acquires, from the action measurement unit 22, the category of the task performed by the caregiver at that location. The model generation unit 26 acquires the location to which the caregiver moved from the location measurement unit 24. The model generation unit 26 acquires, from the time measurement unit 25, the time during which the caregiver stayed at the location to which the caregiver moved. That is, the model generation unit 26 acquires the location to which the caregiver moved, the time that the caregiver stayed at that location, and the category of the task that the caregiver performed at that location.

The model generation unit 26 generates a task category estimation model by performing machine learning using the acquired information as input data and ground truth data. The task category estimation model is a trained model configured to accept as input the location to which a caregiver moved and the time that the caregiver stayed at that location, and to output an estimated result of the category of task performed by the caregiver at the location to which the caregiver moved. That is, the task category estimation model is configured to receive location information and time information for a caregiver as input, and to output an estimated result of the category of a task performed by the caregiver. The training data is data that combines the location to which the caregiver moved and the time that the caregiver stayed at that location. The ground truth data is data that indicates the category of the task performed by the caregiver. The model generation unit 26 generates a task category estimation model by performing machine learning using data in which the location information and time information included in the training data are associated with the categories of tasks included in the ground truth data. The model generation unit 26 outputs the generated task category estimation model to the task category estimation unit 30.

In a case in which the caregiver moves to a plurality of locations, the model generation unit 26 may acquire, from the location measurement unit 24, location information including information regarding the plurality of locations to which the caregiver moved and acquire, from the time measurement unit 25, time information including information regarding the time that the caregiver stayed at each of the plurality of locations to which the caregiver moved. Information that combines location information including information about a plurality of locations to which a caregiver moved and time information including information about the time that the caregiver stayed at the plurality of locations to which the caregiver moved is also referred to as movement history information for the caregiver. In other words, in a case in which the caregiver moves to a plurality of locations, the model generation unit 26 can acquire movement history information for the caregiver and the categories of tasks performed by the caregiver at each location included in the movement history information.

The model generation unit 26 may perform machine learning using data that combines the movement history information for the caregiver and the categories of tasks performed by the caregiver at each location included in the movement history information to generate the task category estimation model. The task category estimation model generated in this case may be configured to accept the movement history information for the caregiver as input, and output an estimated result of the category of the task performed by the caregiver at at least one of the plurality of locations, included in the movement history information for the caregiver, to which the caregiver moved. In other words, the task category estimation model is configured to accept as input location information regarding a plurality of locations to which the caregiver moved and time information regarding the time that the caregiver stayed at each location, and to output an estimated result of the category of the task performed by the caregiver at at least one of the plurality of locations.

### <Example of operation of the task category estimation unit 30>

An example of an operation in which the task category estimation unit 30 of the information processing apparatus 10 estimates the category of a task performed by a caregiver using the task category estimation model will be described with reference to FIG. 3.

The location measurement unit 32 acquires the definition of the location to which the caregiver moves from the location definition unit 14. The location measurement unit 32 acquires coordinate information for the caregiver as a measurement result of the location to which the caregiver moved. The location recognition unit 33 recognizes the location of the caregiver as meta information defined by the location definition unit 14, based on the coordinate information for the caregiver acquired by the location measurement unit 32. In other words, the location recognition unit 33 may generate meta information for the location to which the caregiver moved as the recognition result of the caregiver's location. The location recognition unit 33 outputs the recognition result for the location of the caregiver to the estimation unit 35. The location recognition unit 33 may output the recognition result for each of the plurality of locations included in the movement history of the caregiver to the estimation unit 35. The location measurement unit 32 and the location recognition unit 33 may be configured integrally.

The time measurement unit 34 acquires the measurement result of the time that the caregiver stayed at one location or at each of a plurality of locations. The time measurement unit 34 outputs the measurement result of the time that the caregiver stayed at one location or at each of a plurality of locations to the estimation unit 35. The time measurement unit 34 may be configured to be identical or similar to the time measurement unit 25 of the action learning unit 20.

The estimation unit 35 operates the task category estimation model generated by the action learning unit 20. As described above, the task category estimation model is configured to receive input of location information and time information for a caregiver and output an estimated result of the category of a task performed by the caregiver at at least one location. The estimation unit 35 inputs the recognition result of the caregiver's location acquired from the location recognition unit 33 into the task category estimation model as the location information for the caregiver. The estimation unit 35 inputs the measurement result of the time that the caregiver stayed at the location to which the caregiver moved, as acquired from the time measurement unit 34, into the task category estimation model as the time information for the caregiver. The estimation unit 35 outputs the estimated result of the category of the task by the caregiver, as outputted from the task category estimation model, to the output unit 36.

The estimation unit 35 may input information that combines the recognition results of a plurality of locations to which the caregiver moved, as acquired from the location recognition unit 33, and the measurement results of the time that the caregiver stayed at each of the plurality of locations, as acquired from the time measurement unit 34, into the task category estimation model as movement history information for the caregiver. The estimation unit 35 may output, to the output unit 36, the estimated result outputted from the task category estimation model. This estimated result is for the category of the task performed by the caregiver at at least one location included in the movement history information for the caregiver.

In a case in which the caregiver moves to a plurality of locations, the location information for the caregiver included in the movement history information may include information regarding the location at which the caregiver stayed before the location at which the caregiver performed the task for which the category is to be estimated. The location information for the caregiver included in the movement history information may also include information regarding the location at which the caregiver stayed after the location at which the caregiver performed the task for which the category is to be estimated. The time information for the caregiver included in the movement history information may include information regarding the time that the caregiver stayed at the location at which the caregiver stayed before the location at which the caregiver performed the task for which the category is to be estimated. The time information for the caregiver included in the movement history information may also include information regarding the time that the caregiver stayed at the location to which the caregiver moved after the location at which the caregiver performed the task for which the category is to be estimated.

The output unit 36 outputs the estimated result of the category of the task by the caregiver, as acquired from the estimation unit 35, and displays the estimated result on the display apparatus 60 or stores the estimated result in the database 70.

### <Example of estimating category of task>

An example in which the category of a task by the caregiver is estimated by applying the above-described operational example of the information processing apparatus 10 to the following environment will be described.

First, the tasks performed by a total of 15 day- and night-shift caregivers at a care facility were measured by a time and motion study. Specifically, the task start time, task end time, task location, and actions of each caregiver were recorded.

Definition data for classifying the task location into 10 categories was generated in advance. The 10 categories for the task locations were moving, resident's rooms, eating places, living areas, bath rooms, toilets, office rooms, staff rooms, passage, and other areas.

Definition data for classifying tasks performed by caregivers into 35 categories was generated in advance. The 35 categories of the tasks were wake up/sleep, change of position, dressing/cosmetic cleaning, residents' transfer, excretion, eating, cleanliness, environmental maintenance, laundry, going out, recreation, transportation, medical procedure, management of medicine, measurement of vital signs, clinical examination, bodywork, physical therapy, occupational therapy, speech and language therapy, care planning, nutritional management, meal service, hygiene management, employees' transfer, meeting, cleanliness of office, and other work; understanding residents, information sharing, data recording, confirmation, visitor facing, and other service; and break.

Based on the information measured by the time and motion study, the action learning unit 20 generated training data that includes the task location where the caregiver performed the task and the time when the task was performed, along with the locations where the caregiver performed the 10 tasks immediately prior to performing the task and the time that the caregiver stayed at those locations. The action learning unit 20 generated data on the categories of tasks performed by the caregiver as ground truth data used in machine learning to generate a task category estimation model. However, in the present embodiment, the categories of tasks included in the ground truth data generated based on the measurement results of the time and motion study were 25 out of the 35 categories.

As described above, the task category estimation model may accept as input the movement history information for a caregiver and output an estimated result of the category of the task performed by the caregiver at at least one area, included in the movement history information, to which the caregiver moved. In the present embodiment, the task category estimation model estimates the category of a task by a caregiver based on location information regarding the location to which the caregiver moved before the area at which the action to be estimated was performed and time information regarding the time that the caregiver stayed at that location.

The action learning unit 20 performed machine learning on the training data and ground truth data using a random forest, a support vector machine (SVM), or a three-layer convolutional neural network to generate a task category estimation model.

The task category estimation unit 30 estimated the category of the task by the caregiver using the task category estimation model generated as described above. Here, the recognition rate of each model was verified by five-fold cross-validation. The recognition rate is the rate at which the estimated result of the category of a task matches the correct category. In the verification, the task category estimation unit 30 inputted, into the task category estimation model, the location information and time information when a task for which the correct category was known was performed. The task category estimation unit 30 acquired the estimated result of the task category from the task category estimation model and calculated the recognition rate by a comparison with the correct category. As a result, the recognition rate using the model generated by machine learning using the random forest was 41.7 %. The recognition rate using the model generated by machine learning using the SVM was 52.2 %. The recognition rate using the model generated by machine learning using the neural network was 52.5 %.

### <Examples of categories of caregiving-related tasks performed by caregivers>

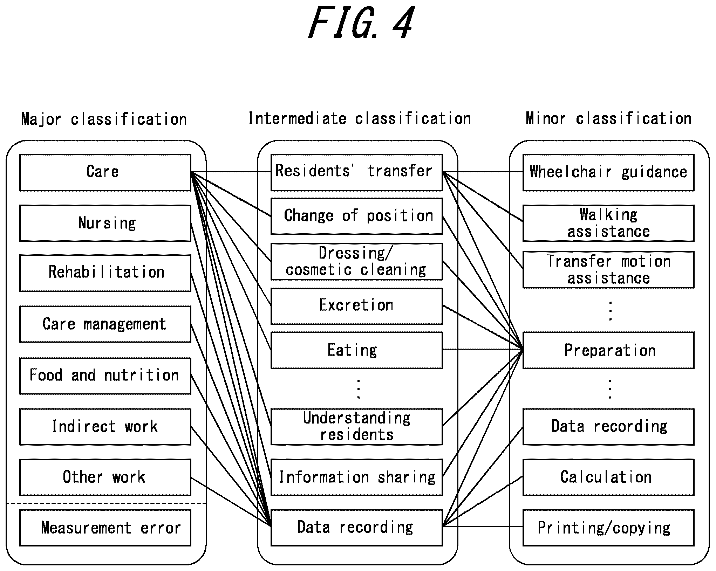

Caregiving-related tasks performed by a caregiver may be classified into categories in a plurality of levels, as illustrated in FIG. 4. In the example in FIG. 4, the categories of caregiving-related tasks performed by the caregiver are divided into three levels, namely major classification, intermediate classification, and minor classification. The major classification includes at least one intermediate classification. In other words, the number of categories belonging to the major classification is equal to or less than the number of categories belonging to the intermediate classification. The intermediate classification includes at least one minor classification. In other words, the number of categories belonging to the intermediate classification is equal to or less than the number of categories belonging to the minor classification. The major classification is also called the first level. The intermediate classification is also called the second level. The minor classification is also called the third level. The second level is included in the first level. In other words, the second level is a lower level than the first level. The third level is included in the second level. In other words, the third level is a lower level than the second level. The number of levels is not limited to three and may instead be two, or may be four or more.

In the present embodiment, the major classification (first level) contains categories that classify the tasks performed by the caregiver by the type of service. The major classification may include, for example, caregiving, nursing, rehabilitation, nursing support, meals and nutrition, indirect duties, tasks other than duties, common duties, or other duties. The major classification may include measurement error as a category for classification when the task performed by the caregiver cannot be classified into any category. The major classification is not limited to these examples and may include various categories.

In the present embodiment, the intermediate classification (second level) includes categories that classify the tasks performed by the caregiver by purpose or situation of the task. The intermediate classification corresponds to the categories that classify tasks into 35 types in the above-described example of category estimation.

As categories that belong to the major classification of care, the intermediate classification may include, for example, wake up/sleep, change of position, dressing/cosmetic cleaning, residents' transfer, excretion, eating, cleanliness, environmental maintenance, laundry, going out, recreation, or transportation. As categories that belong to the major classification of nursing, the intermediate classification may include, for example, medical procedure, management of medicine, measurement of vital signs, clinical examination, bodywork.

As categories that belong to the major classification of rehabilitation, the intermediate classification may include, for example, physical therapy, occupational therapy, or speech and language therapy. As categories that belong to the major classification of care management, the intermediate classification may include, for example, care planning. As categories that belong to the major classification of food and nutrition, the intermediate classification may include, for example, nutritional management, meal service, or hygiene management.

As categories that belong to the major classification of indirect work, the intermediate classification may include, for example, employees' transfer, meeting, and cleanliness of office. As categories that belong to the major classification of out of work, the intermediate classification may include, for example, break. As categories that belong to the major classification of common duties, the intermediate classification may include, for example, understanding residents, information sharing, data recording, confirmation, visitor facing, and other service.

The minor classification (third level) includes categories that classify the tasks performed by the caregiver according to the content of the tasks.

As categories that belong to the intermediate classification of wake up/sleep, the minor classification may include, for example, wake up assistance or sleep assistance. As categories that belong to the intermediate classification of change of position, the minor classification may include, for example, change of position or seat position adjustment. As categories that belong to the intermediate classification of dressing/cosmetic cleaning, the minor classification may include, for example, dressing assistance or cosmetic cleaning assistance. As categories that belong to the intermediate classification of residents' transfer, the minor classification may include, for example, wheel chair guidance, walking assistance, transfer motion assistance, or standing assistance. As categories that belong to the intermediate classification of excretion, the minor classification may include, for example, excretion assistance, napkin change, or hand-wash assistance. As categories that belong to the intermediate classification of eating, the minor classification may include, for example, eating assistance, drinking assistance, delivery of dish, collection of dish, or cooking assistance. As categories that belong to the intermediate classification of cleanliness, the minor classification may include, for example, bath assistance, foot bath assistance, face-wash assistance, bed bath, genital wash, oral care, cleaning the ears, nail care, shaving, shampoo, hand-wash assistance, or gargle assistance. As categories that belong to the intermediate classification of environmental maintenance, the minor classification may include, for example, sheet change, collection of waste, cleaning up residents' room, confirmation of residents' items, temperature control, humidity conditioning, brightness control, or air ventilation. As categories that belong to the intermediate classification of laundry, the minor classification may include, for example, laundry or collection of clothes. As categories that belong to the intermediate classification of going out, the minor classification may include, for example, shopping or shopping escort. As categories that belong to the intermediate classification of recreation, the minor classification may include, for example, body exercise, oral exercise, music, picture-card show, or cooking. As categories that belong to the intermediate classification of transportation, the minor classification may include, for example, transportation or assistance for vehicle ingress/egress.

As categories that belong to the intermediate classification of medical procedure, the minor classification may include, for example, tracheal aspiration, wound care, application of ointment, sterilization, transdermal patch, intravascular pressure monitoring, intravenous infusion, management of gastric fistula, urination management, enema clyster, or oxygen therapy. As categories that belong to the intermediate classification of management of medicine, the minor classification may include, for example, oral drug administration, tubal drug administration, drug delivery, ocular instillation, injection, suppository administration, or availability check. As categories that belong to the intermediate classification of measurement of vital signs, the minor classification may include, for example, measurement of body temperature, measurement of SpO2, measurement of heart rate, measurement of blood pressure, measurement of body weight, or measurement of vital signs. As categories that belong to the intermediate classification of clinical examination, the minor classification may include, for example, attendance at clinical examination or escort to hospital/clinic. As categories that belong to the intermediate classification of bodywork, the minor classification may include, for example, massage or functional recovery.

As categories that belong to the intermediate classification of physical therapy, the minor classification may include, for example, range of motion exercise, heat therapy, gait exercise, standing exercise, stepping exercise, therapeutic exercise, or assistance of physical therapists. As categories that belong to the intermediate classification of occupational therapy, the minor classification may include, for example, occupational task, calculation exercise, cognitive exercise, memory exercise, art work exercise, gaming exercise, or assistance of occupational therapists. As categories that belong to the intermediate classification of rehabilitation, the minor classification may include, for example, speech exercise, auditory comprehensive exercise, swallowing test, indirect swallowing exercise, or direct swallowing exercise.

As categories that belong to the intermediate classification of care planning, the minor classification may include, for example, care planning, conference, monitoring, or assessment. As categories that belong to the intermediate classification of nutritional management, the minor classification may include, for example, nutritional care planning, conference, monitoring, assessment of nutritional care, screening of nutritional care, or consultation of food and nutrition. As categories that belong to the intermediate classification of meal service, the minor classification may include, for example, meal menu planning, cooking, counting meal set, or procurement of ingredient. As categories that belong to the intermediate classification of hygiene management, the minor classification may include, for example, physical condition management, food management, or equipment management.

As categories that belong to the intermediate classification of employees' transfer, the minor classification may include, for example, employees' transfer, waiting, or conveyance. As categories that belong to the intermediate classification of meeting, the minor classification may include, for example, morning meeting, evening meeting, or meeting. As categories that belong to the intermediate classification of cleanliness of office, the minor classification may include, for example, cleaning up common space or arrangement.

As categories that belong to the intermediate classification of understanding residents, the minor classification may include, for example, communicate care, active listening, observation, confirmation of residents' condition, or response to nurse call. As categories that belong to the intermediate classification of information sharing, the minor classification may include, for example, oral information sharing, handing-over, calling, or sending and receiving faxes. As categories that belong to the intermediate classification of data recording, the minor classification may include, for example, data recording, calculation, or printing/copying. As categories that belong to the intermediate classification of confirmation, the minor classification may include, for example, data confirmation, task confirmation, or equipment confirmation. As categories that belong to the intermediate classification of visitor facing, the minor classification may include, for example, family facing or visitor facing.

As common tasks, the minor classification may include preparation or hand-wash.

The action learning unit 20 may generate ground truth data by identifying the category of the task performed by the caregiver based on the categories belonging to the classification of each level described above. As described above, the number of categories belonging to the major classification is smaller than the number of categories belonging to the intermediate classification. Furthermore, the number of categories belonging to the intermediate classification is smaller than the number of categories belonging to the minor classification.

At this point, it is assumed that the amount of information on the actions by the caregiver that the action learning unit 20 can collect using the action measurement unit 22 is constant. When task categories are associated with collected information on the caregiver's actions, the number of pieces of collected information on the caregiver's actions that can be associated with each task category is inversely proportional to the number of task categories. In other words, the fewer the number of task categories classified by the task category estimation model, the greater the amount of training data corresponding to the task categories. The more training data there is, the higher the accuracy of estimating the task category. Therefore, the action learning unit 20 may generate a plurality of data sets by combining input data and ground truth data that differ in the number of task categories that correspond to the collected information on the caregiver's actions, and generate a task category estimation model learned using each of the data sets.

For example, the action learning unit 20 may classify collected information regarding the caregiver's actions into categories belonging to the major classification to generate ground truth data, and perform machine learning using the ground truth data and input data corresponding to the ground truth data to generate a task category estimation model that can estimate the category of the task by the caregiver in a category belonging to the major classification. The action learning unit 20 may classify collected information regarding the caregiver's actions into categories belonging to the intermediate classification to generate ground truth data, and perform machine learning using the ground truth data and input data corresponding to the ground truth data to generate a task category estimation model that can estimate the category of the task by the caregiver in a category belonging to the intermediate classification. The action learning unit 20 may classify collected information regarding the caregiver's actions into categories belonging to the minor classification to generate ground truth data, and perform machine learning using the ground truth data and input data corresponding to the ground truth data to generate a task category estimation model that can estimate the category of the task by the caregiver in a category belonging to the minor classification.

The action learning unit 20 is not limited to the major classification, intermediate classification, or minor classification and may generate a task category estimation model that can estimate the category of a task by the caregiver in various other category groups. For example, the action learning unit 20 may generate a task category estimation model that estimates the task performed by the caregiver to be either a task category that involves movement or a task category that does not involve movement. In the above embodiment, when the tasks were classified into categories limited to tasks involving movement and tasks not involving movement, the recognition rate was increased to 82.8 % by the aforementioned method using a neural network.

The action learning unit 20 may generate a task category estimation model that can estimate the category of a task by the caregiver in one of the major classification, intermediate classification, or minor classification. The action learning unit 20 may generate a task category estimation model that can estimate the category of a task by the caregiver in two or more of the major classification, intermediate classification, or minor classification. For example, the action learning unit 20 may generate a task category estimation model that can estimate the category of a task by the caregiver in both the major classification and the intermediate classification. In this case, the task category estimation model outputs both the estimated result of the category included in the major classification and the estimated result of the category included in the intermediate classification.

### <Estimation using subsequent movement history>

In the above-described embodiment, the task category estimation model was configured to estimate the category of a task by the caregiver based on the location information regarding a location to which the caregiver moved before the location where the task for which the category was to be estimated was performed and the time information regarding the time that the caregiver stayed at that location. That is, the category of a task performed by the caregiver at a certain location was estimated based on a location to which the caregiver moved in the past.

The task category estimation model may be configured to estimate the category of a task by the caregiver based on the location information regarding a location to which the caregiver moved after the location where the task for which the category was to be estimated was performed and the time information regarding the time that the caregiver stayed at that location. In other words, the category of the task performed by the caregiver at a certain location may be estimated based on the location to which the caregiver will move in the future.

As an example of this case, the action learning unit 20 generated training data that, based on information measured by a time and motion study, includes information that combines location information indicating the task location where the task for which the category was to be estimated was performed and time information indicating the task time at which the task was performed, along with information that combines location information regarding the locations where five tasks were performed immediately before performance of the task for which the category was to be estimated and time information indicating the time when those tasks were performed, and information that combines location information regarding the locations where five tasks were performed immediately after performance of the task for which the category was to be estimated and time information indicating the time when those tasks were performed. As ground truth data to be used in machine learning for generating a task category estimation model, the action learning unit 20 generated the categories of tasks performed by the caregiver at locations to which the caregiver moved, as identified by the location information included in the training data. The action learning unit 20 generated the task category estimation model by performing machine learning using the training data and ground truth data.

The task category estimation unit 30 estimated the category of the task by the caregiver using the task category estimation model generated as described above. Here, the recognition rate of each model was verified by five-fold cross-validation. The recognition rate using the model generated by machine learning using a neural network was 55.6 %. The recognition rate using a model generated by machine learning using a neural network based on the locations the caregiver moved to in the past was 52.5 %. Therefore, the recognition rate can be improved by using a model generated based on not only past locations of the caregiver but also future locations of the caregiver.

On the other hand, when the category of a task performed by the caregiver is estimated using a model generated based on the areas to which the caregiver moved in the past, the category can be estimated in real time or near real time by measuring the locations to which the caregiver moved and the time the caregiver stays at each location.

### <Generating ground truth data through data assimilation>

In the above embodiment, the categories of tasks included in the ground truth data generated based on the measurement results of the time and motion study were 25 out of the 35 categories. In other words, there were 10 task categories that were not included in the ground truth data. In a case in which some task categories are not included in the ground truth data, a model generated by performing machine learning using that ground truth data is unlikely to be configured to output, as the estimated result, the task categories that are not included in the ground truth data. Furthermore, a model generated by performing machine learning using the ground truth data might not output, as the estimated result, task categories that are not included in the ground truth data.

Task categories that are not included in the measurement results of the time and motion study could be tasks that occur rarely. Task categories that occur rarely may include, for example, a category of tasks that are performed when a disaster or accident occurs. It is difficult to collect measurements with a time and motion study for categories of tasks that occur rarely.

Therefore, the action learning unit 20 may use a technique called data assimilation to generate training data corresponding to ground truth data that includes categories of tasks that are not included in the measurement results from the time and motion study. Data assimilation is a method for generating training data or ground truth data through simulation. A model generated by performing machine learning using the training data or ground truth data generated in this manner can be configured to output, as an estimated result, even rarely occurring task categories that are not included in the measurement results of a time and motion study.

### <Examples of other items included in the training data>

The training data may include other items. The training data may include, for example, nurse call information, monitoring sensor information, care equipment logs, task manual information, or the like. The training data may include seasonal variation or calendar information. The training data may include climate data such as weather or temperature. The training data may include information for the caregiver, other than the location information and time information for the caregiver, that can be acquired using an IoT (Internet of Things) device.

### <Description of the care process>

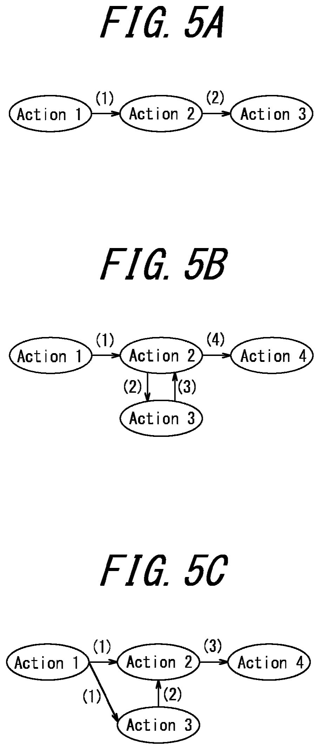

The tasks performed by a caregiver in a care service can be described as a care process that combines a plurality of actions. A care process can be described as an independent process in which a caregiver performs a plurality of actions one by one in sequence, for example as illustrated in FIG. 5A. In FIG. 5A, the caregiver (1) progresses from "Action 1" to "Action 2", and (2) progresses from "Action 2" to "Action 3".

In addition to being an independent process, a care process can also be described as a parallel process in which a caregiver performs a plurality of actions in parallel. A parallel process can be described as a process in which some actions are parallel, for example as illustrated in FIG. 5B. In FIG. 5B, the caregiver (1) progresses from "Action 1" to "Action 2", (2) and (3) performs "Action 2" and "Action 3" in parallel, and (4) progresses from "Action 2" or "Action 3" to "Action 4".

A parallel process can be described as a process in which first actions being parallel, for example as illustrated in FIG. 5C. In FIG. 5C, the caregiver (1) progresses from "Action 1" to "Action 2" and "Action 3" in parallel, (2) performs "Action 2" and "Action 3" in parallel, and (3) progresses from "Action 2" to "Action 4."

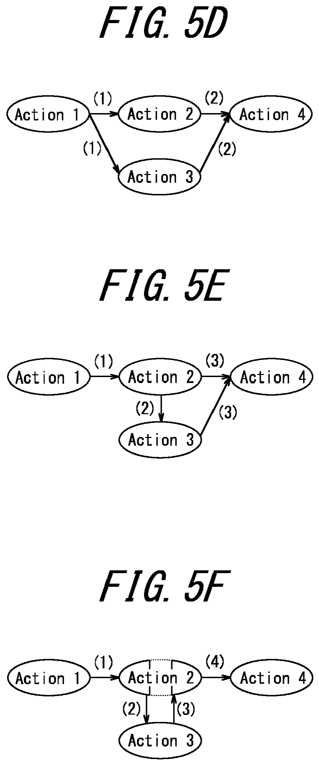

A parallel process can be described as a process in which all actions are parallel, for example as illustrated in FIG. 5D. In FIG. 5D, the caregiver (1) progresses from "Action 1" to "Action 2" and "Action 3" in parallel, performing "Action 2" and "Action 3" in parallel, and (2) progresses from "Action 2" and "Action 3" to "Action 4."

A parallel process can be described as a process in which last actions being parallel, for example as illustrated in FIG. 5E. In FIG. 5E, the caregiver (1) progresses from "Action 1" to "Action 2", (2) performs "Action 2" and "Action 3" in parallel, and (3) progresses from "Action 2" and "Action 3" in parallel to "Action 4".

The care process can also be described as an interruption process, illustrated in FIG. 5F, in which the caregiver temporarily puts a particular action on hold to perform another action and then resumes the original action. In FIG. 5F, the caregiver (1) proceeds from "Action 1" to "Action 2", (2) interrupts "Action 2" to perform "Action 3", (3) after completing "Action 3", resumes the interrupted "Action 2", and (4) proceeds from "Action 2" to "Action 4".

The action learning unit 20 may generate movement history information for a caregiver as training data, taking into account the flow of actions in these care processes, generate task categories corresponding to the training data as ground truth data, and generate a task category estimation model by performing machine learning using the ground truth data. The task category estimation model may be configured to output the estimated result of the category of a task including some of the actions included in a care process. The task category estimation model may be configured to treat the entire care process as one task and output the estimated result of the category of that task.

In a case in which each action in a care process is performed at a different location, the action learning unit 20 may generate movement history information, as training data, including the order of movement to the location where each action is performed, generate a task category corresponding to the training data as ground truth data, and generate a task category estimation model by performing machine learning using the ground truth data. In this case, the task category estimation model is configured to treat the entire care process as one task and output an estimated result of the task category.

### <Example of an estimated result>

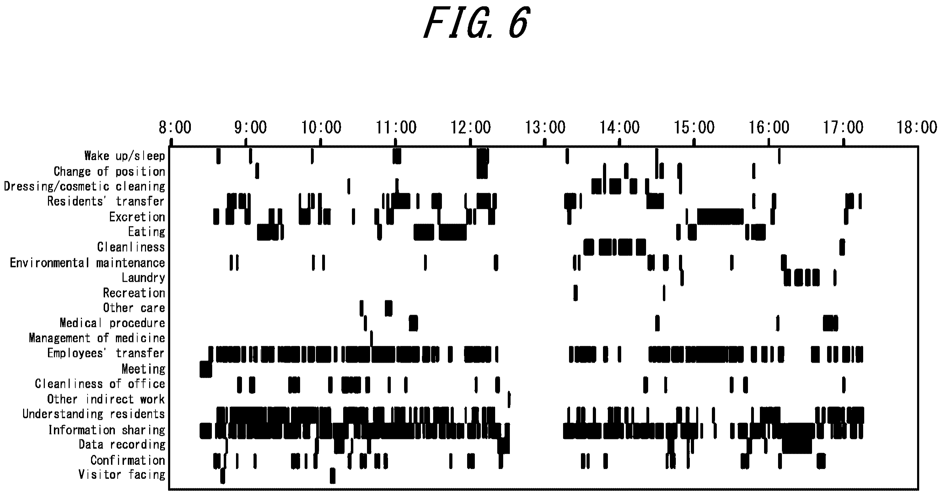

As described above, the output unit 36 of the task category estimation unit 30 may display, on the display apparatus 60, the estimated result of the category of the task performed by the caregiver. The display apparatus 60 may display the estimated result in the form of a timeline associated with the time periods during which the caregiver is estimated to have performed a task in each category, as illustrated in FIG. 6. The horizontal axis in FIG. 6 represents the passage of time from left to right. The time periods during which a task in each category was performed are illustrated as black rectangles. By displaying in this manner, the estimated result of the category of the task by the caregiver can be easily understood.

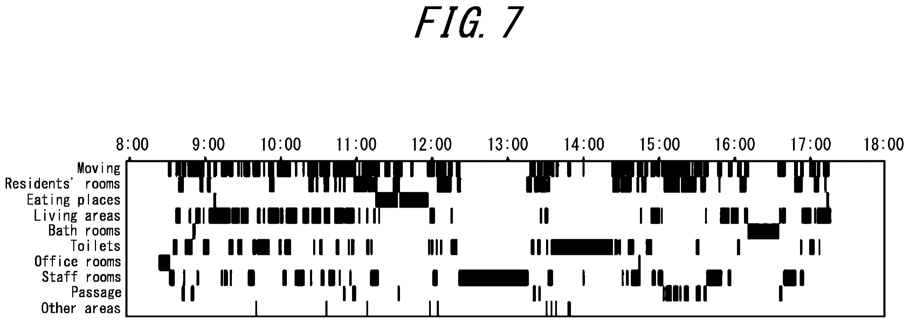

The display apparatus 60 may display the time periods during which the caregiver stayed at each location in the form of a timeline, as illustrated in FIG. 7. The horizontal axis in FIG. 7 represents the passage of time from left to right. The time periods during which the caregiver stayed at each location are illustrated as black rectangles.

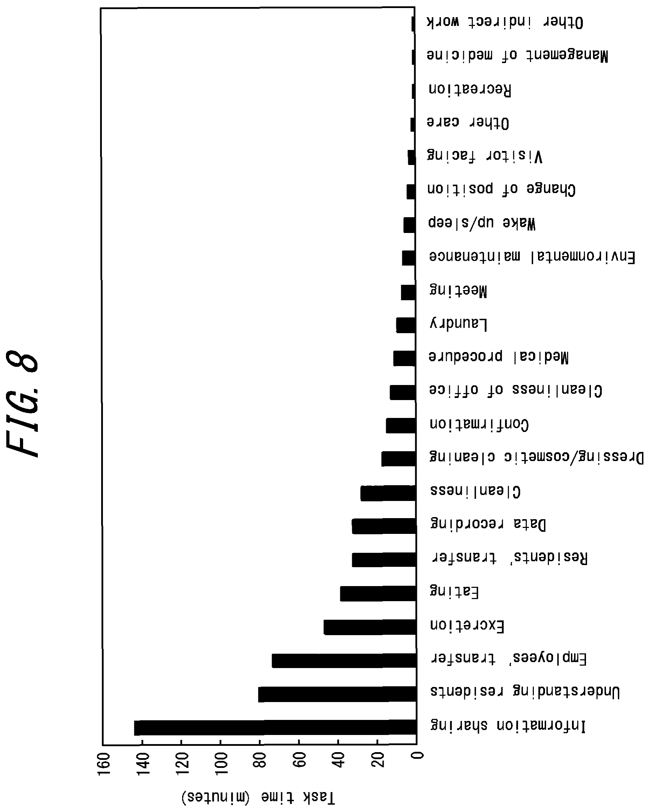

As illustrated in FIG. 8, the display apparatus 60 may display, as a bar graph, the total length of time that the caregiver is estimated to have performed a task in each category. The horizontal axis of the graph in FIG. 8 corresponds to the task category. The vertical axis represents the length of time that the caregiver is estimated to have performed a task in each category. In a case in which a task in one category is performed multiple times, the estimated length of time for performing the task in that category is the sum of the time for performing the task each time. The display apparatus 60 may also display, as a histogram, the frequency of the task, in each category, that is estimated to have been performed by the caregiver. In other words, the display apparatus 60 may display statistical data on the categories of tasks that are estimated to have been performed by the caregiver. By displaying in this manner, the estimated result of the category of the task by the caregiver can be easily understood.

In a case in which the task category estimation unit 30 performs estimation in real time using the estimation unit 35, the display apparatus 60 may change the display content in real time according to the real-time estimated result. The display apparatus 60 may display the estimated result of the categories of tasks performed by a plurality of caregivers in a comparable format. The display apparatus 60 may display, in a comparable format, the category of a task performed by the caregiver as estimated based on the location information and time information acquired before changing the procedure of the task performed by the caregiver, and the category of the task performed by the caregiver as estimated based on location information and time information acquired after changing the procedure of the task performed by the caregiver. The display apparatus 60 is not limited to these examples and may display the estimated result of the category of a task performed by the caregiver in various other forms. The output unit 36 of the task category estimation unit 30 may output the content displayed on the display apparatus 60 as data or information to another device such as the database 70.

### <Example flowchart>

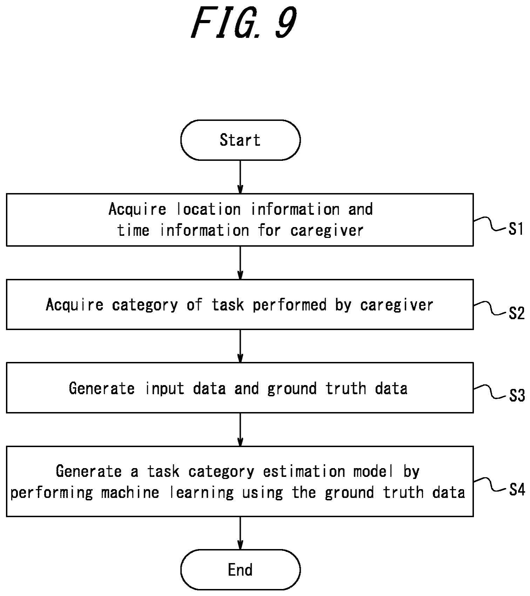

The action learning unit 20 of the information processing apparatus 10 may execute a model generation method including the steps of the flowchart illustrated in FIG. 9. The model generation method may be realized as a model generation program executed by a processor constituting the action learning unit 20. The model generation program may be stored on a non-transitory computer readable medium.

The action learning unit 20 uses the location measurement unit 24 and the time measurement unit 25 to acquire the location information and time information for the caregiver (step S1). The action learning unit 20 uses the action measurement unit 22 to acquire the category of the task performed by the caregiver (step S2). The action learning unit 20 generates input data including the location information and time information for the caregiver and ground truth data including the categories of tasks performed by the caregiver at each location (step S3). The action learning unit 20 generates a task category estimation model by performing machine learning in the model generation unit 26 using the input data and ground truth data (step S4). The action learning unit 20 outputs the generated task category estimation model to the task category estimation unit 30. After executing the procedure of step S4, the action learning unit 20 ends the execution of the procedure of the flowchart in FIG. 9.

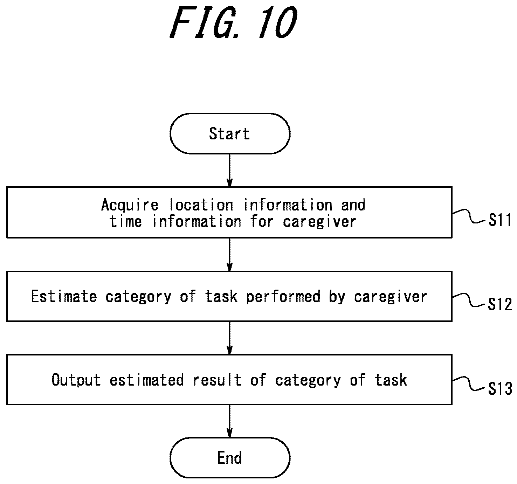

The task category estimation unit 30 of the information processing apparatus 10 may perform a task category estimation method including the steps of the flowchart illustrated in FIG. 10. The task category estimation method may be realized as a task category estimation program executed by a processor constituting the task category estimation unit 30. The task category estimation program may be stored in a non-transitory computer readable medium.

The task category estimation unit 30 uses the location measurement unit 32 and the time measurement unit 34 to acquire location information and time information for the caregiver (step S11). Via the estimation unit 35, the task category estimation unit 30 uses the task category estimation model to estimate the category of the task performed by the caregiver (step S12). Via the output unit 36, the task category estimation unit 30 outputs the estimated result of the category of the task performed by the caregiver (step S13). After executing the procedure of step S13, the task category estimation unit 30 ends the execution of the procedure of the flowchart in FIG. 10.

### (Summary)

As described above, the information processing system 1 of the present embodiment can estimate the category of a caregiving-related task performed by caregiver at a caregiving site, such as a care facility, by inputting the location information and time information for the caregiver into a task category estimation model and acquiring the estimated result of the category of the task from the task category estimation model. By using the task category estimation model, the information processing system 1 can collect task data on caregivers without the need for human labor by an observer or the like. As a result, the task data on the caregiver is collected efficiently. Efficient collection of task data on caregivers efficiently supports the use of task data on caregivers to improve care services.

While embodiments of the present disclosure have been described with reference to the drawings and examples, it should be noted that various modifications and revisions may be implemented by those skilled in the art based on the present disclosure. Accordingly, such modifications and revisions are included within the scope of the present disclosure. For example, functions or the like included in each component, each step, or the like can be rearranged without logical inconsistency, and a plurality of components, steps, or the like can be combined into one or divided. Although the embodiments according to the present disclosure have been described mainly in terms of an apparatus, the embodiments according to the present disclosure may also be realized as a method including steps executed by each component of an apparatus. Embodiments according to the present disclosure can also be realized as a method or program executed by a processor included in an apparatus, or as a storage medium having the program recorded thereon. Such embodiments are also to be understood as included in the scope of the present disclosure.

### REFERENCE SIGNS LIST

1 Information processing system
10 Information processing apparatus (12: task category definition unit, 14: location definition unit)
20 Action learning unit (22: action measurement unit, 24: location measurement unit, 25: time measurement unit, 26: model generation unit)
30 Task category estimation unit (32: location measurement unit, 33: location recognition unit, 34: time measurement unit, 35: estimation unit, 36: output unit)
40 Sensor
50 Input apparatus
60 Display apparatus
70 Database

## Claims

1. A task category estimation method of estimating a category of a task performed by a caregiver at a caregiving site, the task category estimation method comprising:
acquiring, by an information processing apparatus, location information regarding a plurality of locations to which the caregiver moved at the caregiving site and time information regarding a time that the caregiver stayed at each of the plurality of locations to which the caregiver moved;
estimating, by the information processing apparatus, a category of a task performed by the caregiver at at least one location among the plurality of locations to which the caregiver moved, based on the location information and the time information; and
outputting an estimated result of the category of the task performed by the caregiver.

2. The task category estimation method according to claim 1, wherein in the acquiring of the time information, the time that the caregiver stayed at each of the plurality of locations is acquired using a sensor.

3. The task category estimation method according to claim 1, wherein the time information includes a length of time that the caregiver stayed at at least one of the plurality of locations.

4. The task category estimation method according to claim 1, wherein the location information for the caregiver includes a category of a task that the caregiver can perform at at least one of the plurality of locations.

5. The task category estimation method according to claim 1, wherein the location information for the caregiver includes information on equipment installed in at least one of the plurality of locations.

6. The task category estimation method according to claim 1, wherein the location information for the caregiver includes information on a location at which the caregiver stayed before the location at which the caregiver performed the task to be estimated.

7. The task category estimation method according to claim 1, wherein the location information for the caregiver includes information on a location at which the caregiver stayed after the location at which the caregiver performed the task to be estimated.

8. The task category estimation method according to claim 1, wherein in the estimating of the category of the task performed by the caregiver, the information processing apparatus inputs the location information and the time information into a task category estimation model, and acquires an estimated result, outputted from the task category estimation model, of the category of the task performed by the caregiver.

9. The task category estimation method according to claim 8, further comprising performing, by the information processing apparatus, learning using training data including the location information and the time information, and ground truth data including categories of tasks performed by the caregiver, to generate the task category estimation model.

10. The task category estimation method according to any one of claims 1 to 9, wherein in the outputting of the estimated result of the category of the task performed by the caregiver, the information processing apparatus displays the estimated result of the category of the task performed by the caregiver in a form of a timeline corresponding to a time period during which the caregiver performed the task.

11. The task category estimation method according to any one of claims 1 to 9, wherein in the outputting of the estimated result of the category of the task performed by the caregiver, the information processing apparatus outputs statistical data on the category of the task performed by the caregiver.

12. The task category estimation method according to any one of claims 1 to 9, wherein
the task performed by the caregiver is classified into at least two levels including a first level and a second level below the first level, and
in the estimating of the category of the task performed by the caregiver, the information processing apparatus estimates the category of the task based on at least one of classification into the first level and classification into the second level.

13. The task category estimation method according to claim 12, wherein in the estimating of the category of the task performed by the caregiver, the information processing apparatus estimates the category of the task based on both classification into the first level and classification into the second level.

14. The task category estimation method according to claim 12, wherein
the task performed by the caregiver is classified into the first level by type of care service, and
the task performed by the caregiver is classified into the second level by purpose or situation.

15. The task category estimation method according to claim 14, wherein
the task performed by the caregiver is further classified into a third level below the second level, and
the task performed by the caregiver is classified into the third level by content of the task performed by the caregiver.

16. A task category estimation program configured to cause an information processing apparatus to execute the task category estimation method according to any one of claims 1 to 9.

17. An information processing apparatus comprising a processor configured to execute the task category estimation method according to any one of claims 1 to 9.
